# FASCICULE DE BREVET EUROPEEN

(11) **EP 2 094 312 B1**
(45) Date de publication et mention de la délivrance du brevet: **10.03.2010**
(21) Numéro de dépôt: 07821821.1
(22) Date de dépôt: 25.10.2007
(51) Int. Cl.: A61L 2/04, A61L 2/10, B29C 49/64, B29C 49/68, A61L 2/20

(54) **FOUR ET INSTALLATION POUR LA FABRICATION DE RECIPIENTS STERILES A PARTIR DE PREFORMES EN MATIERE THERMOPLASTIQUE DECONTAMINEES**
OFEN UND ANLAGE ZUR HERSTELLUNG VON STERILEN GEFÄSSEN AUS DEKONTAMINIERTEN VORFORMEN EINES THERMOPLASTISCHEN MATERIALS
FURNACE AND EQUIPMENT FOR PRODUCING STERILE VESSELS FROM DECONTAMINATED PREFORMS OF A THERMOPLASTIC MATERIAL

(30) Priorité: 26.10.2006 FR 0654545
(43) Date de publication de la demande: 02.09.2009
(73) Titulaire: Sidel Participations, 76930 Octeville Sur Mer (FR)
(72) Inventeur: ADRIANSENS, Eric, 76930 Octeville Sur Mer (FR)
(74) Mandataire: Thibaudeau, David A. C. R.
(86) Numéro de dépôt international: PCT/EP2007/061458
(87) Numéro de publication internationale: WO 2008/049876

(56) Documents cités:
- EP-A1- 0 411 970
- WO-A-99/51497
- FR-A- 2 872 734
- FR-A1- 2 195 551
- FR-A1- 2 464 816
- US-B1- 6 183 691
- US-B1- 6 517 776

## Description

L'invention concerne un four et une installation pour la fabrication de récipients stériles à partir de préformes en matière thermoplastiques décontaminées.

La présente invention concerne d'une façon générale le domaine de la stérilisation (ou décontamination) des préformes en matière thermoplastique, notamment en Polyéthylènetéréphtalate (PET), qui sont destinées à la fabrication de récipients, tels que des bouteilles, flacons ou analogues, par un processus de soufflage ou d'étirage-soufflage d'une préforme préalablement conditionnée thermiquement dans un four.

L'invention concerne, selon un de ses aspects, un four de conditionnement thermique de préformes en matière thermoplastique destinées à la fabrication de récipients à corps creux par soufflage ou étirage-soufflage, comportant un dispositif de transport des préformes qui sont mises en rotation sur elle-même et convoyées successivement entre au moins :
- une première zone, dite de chauffage de pénétration, comportant des premiers moyens de chauffage par rayonnement infrarouge destinés à préchauffer le corps de chaque préforme jusqu'à une première température de consigne ;
- une deuxième zone intermédiaire, dite de temporisation qui, interposée sur le trajet des préformes entre la première zone de chauffage et une troisième zone de chauffage, est parcourue par chaque préforme en un laps de temps déterminé ;
- la troisième zone, dite de chauffage de distribution, comportant des seconds moyens de chauffage par rayonnement infrarouge destinés à chauffer le corps de chaque préforme, selon un profil de chauffe déterminé, jusqu'à une deuxième température de consigne.

On connaît de nombreux fours de ce type en particulier pour le chauffage par rayonnement infrarouge d'une préforme afin de la conditionner thermiquement en vue de son moulage, c'est à dire sa transformation par soufflage en un récipient, par exemple une bouteille, un tel four étant alors intégré dans une installation de fabrication dont il constitue l'un des principaux postes.

Les documents FR-A-2872734, EP-A-0.620.099 ou EP-A-0.564.354 décrivent respectivement des exemples de four de conditionnement thermique du corps d'une préforme destinée à la fabrication d'un récipient.

De manière connue, les préformes destinées à la fabrication de récipients stériles ou décontaminés dans une installation sont avantageusement traitées en début du processus de fabrication des récipients, plutôt qu'en fin de processus de fabrication une fois le récipient obtenu par soufflage, car on réduit ainsi la quantité de produit stérilisant utilisée qui est en partie fonction de la surface à décontaminer.

L'invention concerne, selon un autre de ses aspects, une installation pour la fabrication de récipients stériles, à partir d'une préforme en matière thermoplastique décontaminée, qui comporte généralement au moins :
- un dispositif d'alimentation en préformes ;
- un poste de conditionnement thermique comportant un dispositif de transport de préformes qui sont mises en rotation sur elle-même et convoyées à travers au moins un four destiné à chauffer chaque préforme en vue de son moulage ; et
- un poste de moulage comportant au moins un moule et un dispositif de soufflage associé destiné à transformer chaque préforme en un récipient à corps creux.

On connaît de nombreuses installations pour la fabrication de récipients stériles de ce type par soufflage ou étirage-soufflage et comportant de plus des moyens de stérilisation de ces préformes, par exemple par voie chimique en procédant notamment au mouillage des surfaces à décontaminer par trempage dans une solution stérilisante, telle que du peroxyde d'hydrogène (H₂O₂).

Le document WO-A-99/03667 décrit par exemple un procédé pour la fabrication de récipients stériles et une installation pour la mise en oeuvre de ce procédé.

L'installation représentée à la figure 5 de ce document comporte principalement un dispositif d'alimentation en préformes, un poste de conditionnement thermique comportant un dispositif de transport de préformes à travers au moins un four destiné à chauffer chaque préforme en vue de son moulage et un poste de moulage comportant au moins un moule et un dispositif de soufflage associé destiné à transformer chaque préforme en un récipient final à corps creux.

Dans cette installation, les préformes sont traitées par des moyens de stérilisation, en amont du four de chauffage de la préforme, de manière à décontaminer la surface cylindrique de la paroi interne de la préforme.

La paroi interne de la préforme correspond à la paroi interne du récipient à corps creux destiné à être fabriqué par soufflage à partir de cette préforme de sorte que cette paroi interne de la préforme délimite le volume intérieur du récipient destiné à être rempli ultérieurement, par exemple par un liquide donné dans le cas d'une bouteille ou d'un flacon.

Les moyens de stérilisation décrits dans ce document comprennent notamment un produit de stérilisation, tel qu'une solution de peroxyde d'hydrogène (H₂O₂), qui est activé thermiquement par la chaleur des moyens de chauffage du four, avant d'être éliminé par évaporation.

Les moyens de stérilisation comportent pour ce faire un pulvérisateur constitué par un pistolet de vaporisation permettant de mouiller l'intérieur des préformes avec du produit de stérilisation "froid", c'est-à-dire n'ayant pas été préalablement chauffé et se trouvant donc à l'état liquide.

Le pistolet est typiquement un pistolet de type bi-fluide comportant une buse de liquide et une buse d'air formant un ensemble de projection circulaire qui est susceptible d'être placé au-dessus du trajet des préformes de manière à pulvériser un brouillard de produit de stérilisation.

Le brouillard de produit de stérilisation est formé d'un nuage de gouttelettes qui est projeté par le pistolet vers l'intérieur de la préforme suivant un écoulement de type turbulent.

On a constaté qu'un tel écoulement turbulent de produit de stérilisation à l'intérieur de la préforme conduit à la formation d'un ensemble de gouttelettes qui ne sont pas reparties de manière homogène sur la paroi interne de la préforme.

En effet, l'utilisation d'un pistolet de pulvérisation est notamment caractérisé par un débit important de produit stérilisant qui est obtenu en comprimant un gaz, tel que de l'air comprimé à des pressions d'environ 2 à 3 bars, de sorte qu'il produit un écoulement turbulent.

Or, l'écoulement turbulent conduit au dépôt non homogène de gouttelettes résiduelles de produit stérilisant sur les parois internes des préformes. De plus, les gouttelettes de produit stérilisant forment un excédent qui n'est pas entièrement vaporisé lors du chauffage.

Ces gouttelettes de produit stérilisant provoquent donc d'une part localement une attaque chimique du matériau de la préforme, généralement en polyéthylène téréphtalate (PET), et, d'autre part, lors du chauffage des préformes, produisent un effet loupe sur les rayonnements thermiques de chauffage, ce qui provoque l'apparition de taches sur les parois des récipients, tels que les bouteilles, issues des préformes traitées.

L'apparition de telles taches sur les parois des bouteilles, est un défaut d'aspect du produit, qui est encore parfois appelé "aspect peau d'orange".

De plus, la quantité de solution de stérilisation susceptible d'être utilisée est limitée en raison des obligations légales d'élimination de la solution de stérilisation dont il ne doit subsister tout au plus que de très infimes traces sur le récipient stérilisé et cela afin notamment que le liquide destiné à remplir le récipient ne présente pas une quantité de solution de stérilisation supérieure au taux légal admis.

Par conséquent, les récipients fabriqués conformément à ce procédé ne donnent pas entière satisfaction et, de surcroît, ne présentent pas un degré de décontamination suffisant par rapport au niveau de stérilité requis pour certaines applications industrielles.

En effet, la décontamination opérée dans l'installation selon ce procédé concerne uniquement la paroi interne de la préforme. Cependant, la surface externe du corps de la préforme comporte de manière analogue des germes, bactéries ou spores qui peuvent contaminer les préformes (ou les récipients) ainsi que l'installation de fabrication en général.

Or, les germes, bactéries etc. présents sur la surface externe du corps de la préforme sont susceptibles de se propager dans l'installation, en utilisant notamment comme vecteur le flux d'air de refroidissement circulant autour des préformes dans le four ou encore en contaminant la surface des moules du poste de soufflage de l'installation.

Il existe par conséquent un risque de contamination, encore parfois appelé "risque de contamination croisée", selon lequel la présence de germes, etc. sur la surface externe de la préforme est susceptible d'engendrer des contaminations pouvant affecter la stérilité, du récipient final ou de lots entiers de récipients, notamment la stérilité interne.

C'est l'une des raisons pour lesquelles, des solutions permettant tout particulièrement une décontamination efficace de la surface externe du corps de chaque préforme ont été recherchées.

Une solution possible consiste à traiter la surface externe du corps de la préforme par voie chimique de manière analogue à la paroi interne, c'est-à-dire par pulvérisation, en amont du four de conditionnement thermique, d'une solution de stérilisation, telle que de peroxyde d'hydrogène (H₂O₂), sur la surface externe du corps de la préforme.

Des essais ont toutefois démontré que le flux d'air de refroidissement circulant dans le four provoque l'élimination quasi systématique et immédiate de la majeure partie de la solution de stérilisation appliquée sur la surface externe et cela avant que la solution stérilisante n'ait pu être activée thermiquement par les moyens de chauffage, de sorte que la décontamination de la surface externe du corps de la préforme ainsi obtenue est généralement très faible et donc insuffisante pour atteindre un degré final de décontamination élevé.

L'invention vise à remédier à ces inconvénients en proposant, un nouveau four permettant notamment, selon un procédé de stérilisation global d'une préforme, de fabriquer dans une installation de fabrication de récipients stériles par soufflage ou par étirage-soufflage de préformes, de tels récipients stériles de manière simple, fiable et économique à partir de préformes intégralement stérilisées ou décontaminées, tant à l'intérieur qu'à l'extérieur.

L'invention vise tout particulièrement, mais non exclusivement, la stérilisation de la surface externe du corps d'une préforme, c'est-à-dire l'abaissement du taux de contamination par des germes, bactéries ou spores etc. de cette surface en vue de limiter tout particulièrement le risque de contamination croisée.

Dans ce but, l'invention propose un four de conditionnement thermique de préformes en matière thermoplastique, notamment destiné à équiper une installation de fabrication de récipients stériles par soufflage ou par étirage-souffage, du type comportant un dispositif de transport des préformes qui sont mises en rotation sur elle-même et convoyées successivement entre au moins :
- une première zone, dite de chauffage de pénétration, comportant des premiers moyens de chauffage par rayonnement infrarouge destinés à préchauffer le corps de chaque préforme jusqu'à une première température de consigne ;
- une deuxième zone intermédiaire, dite de temporisation qui, interposée sur le trajet des préformes entre la première zone de chauffage et une troisième zone de chauffage, est parcourue par chaque préforme en un laps de temps déterminé ;
- la troisième zone, dite de chauffage de distribution, comportant des seconds moyens de chauffage par rayonnement infrarouge destinés à chauffer le corps de chaque préforme, selon un profil de chauffe déterminé, jusqu'à une deuxième température de consigne,
**caractérisé en ce que** le four comporte des moyens de stérilisation par émission de rayons ultraviolets qui sont agencés dans la deuxième zone intermédiaire de temporisation de manière à stériliser au moins la surface externe du corps de la préforme jusqu'à un degré de décontamination déterminé.

Selon d'autres caractéristiques du four selon l'invention :
- le four comporte des moyens réfléchissants, tels que des réflecteurs, qui sont agencés dans la deuxième zone intermédiaire de temporisation en vis-à-vis des moyens de stérilisation par ultraviolets, tels que des lampes, de manière à réfléchir sélectivement les rayons ultraviolets émis en direction des préformes circulant entre les moyens de stérilisation et les moyens réfléchissants ;
- le four comporte un dispositif de refroidissement des préformes par circulation d'un flux d'air autour des préformes,
**caractérisé en ce que** les rayons ultraviolets émis par les moyens de stérilisation dans la deuxième zone intermédiaire de temporisation stérilisent l'air circulant autour des préformes ;
- le dispositif de transport comporte des moyens de préhension du col de chaque préforme comportant au moins un noyau interne qui pénètre axialement à l'intérieur du col de la préforme de manière que les rayons ultraviolets émis par les moyens de stérilisation irradient intégralement le col de la préforme.

L'invention propose encore une installation du type décrit précédemment pour la fabrication de récipients stériles à partir d'une préforme en matière thermoplastique décontaminée suivant le procédé de stérilisation, **caractérisée en ce que** l'installation comporte un four de conditionnement thermique comportant :
- des premiers moyens de chauffage destinés à décontaminer au moins la surface externe du corps de la préforme en préchauffant le corps de chaque préforme jusqu'à une première température de consigne ;
- des moyens de stérilisation par émission de rayons ultraviolets destinés à décontaminer au moins la surface externe du corps de la préforme par irradiation de la la préforme (12) exposée pendant un laps de temps (Δt) déterminé aux rayons ultraviolets (UV) émis par les moyens de stérilisation (76) ; et
- des seconds moyens de chauffage destinés à décontaminer au moins la surface externe du corps de la préforme en chauffant le corps de chaque préforme, jusqu'à une deuxième température de consigne,
   de manière à stériliser au moins la surface externe du corps de la préforme jusqu'à un degré de décontamination déterminé.

Avantageusement, l'installation comporte, en amont du four de conditionnement thermique, un poste de stérilisation comportant un dispositif de projection de produit stérilisant pourvus d'au moins une buse apte à projeter, sous forme d'un jet de vapeur, un flux de produit stérilisant vers l'ouverture du col de chaque préforme, dont la température est inférieure à la température de condensation du produit stérilisant, de manière à déposer par condensation sur au moins la paroi interne de la préforme un film de buée de produit stérilisant sensiblement uniforme destiné à stériliser la paroi interne.

Avantageusement, la première température de préchauffage et/ou la deuxième température de chauffage du corps de la préforme sont supérieures ou égales à la température d'activation du produit stérilisant de manière à activer par chauffage le produit stérilisant pour stériliser la paroi interne de la préforme.

Avantageusement, la première température de préchauffage et/ou la deuxième température de chauffage du corps de la préforme sont supérieures à la température d'évaporation du produit stérilisant de manière à éliminer par évaporation le produit stérilisant activé par chauffage.

D'une manière générale, on rappellera que dans le domaine visé par l'invention, le niveau de stérilité d'une préforme
ou d'un récipient s'apprécie en fonction du nombre de germes, bactéries ou spores, de sorte que l'on se réfère le plus souvent au degré de décontamination obtenu.

Le niveau de stérilité ou degré de décontamination est déterminé par comptage de la quantité de germes, etc. qui sont dénombrés après des opérations de lavage, de filtration et de mise en culture et il est généralement exprimé en "Log" en fonction de la réduction logarithmique du nombre de germes, etc.

A titre d'exemple, la réduction logarithmique du nombre de germes est dite de l'ordre de 3 Log, ou encore 3D, lorsque le degré de décontamination correspond à une réduction de 1000 unités (10³).

Bien entendu, le niveau de stérilité est déterminé en fonction des applications et le degré obtenu étant proportionnel à la réduction logarithmique du nombre de germes, etc., on considère le plus souvent un degré de décontamination de l'ordre de 1 Log comme faible voir négligeable et, par comparaison, un degré de décontamination de l'ordre de 6 Log comme particulièrement élevé.

D'autres caractéristiques et avantages de l'invention apparaîtront à la lecture de la description détaillée qui suit pour la compréhension de laquelle on se reportera aux dessins annexés dans lesquels :
- la figure 1 est une vue de dessus qui représente schématiquement un four de conditionnement thermique selon l'invention ;
- la figure 2 est une vue en coupe du four de la figure 1 selon la section 2-2 qui représente schématiquement les première et troisième zones du four pour le chauffage par infrarouge et la stérilisation des préformes ;
- la figure 3 est une vue en coupe du four de la figure 1 dans la deuxième zone de temporisation selon la section 3-3 et représente schématiquement un exemple d'implantation des moyens de stérilisation par ultraviolets comportant des réflecteurs associés ;
- la figure 4 est une vue analogue à la figure 3 qui illustre une variante de réalisation selon laquelle les moyens de stérilisation par ultraviolets sont agencés de part et d'autre des préformes ;
- la figure 5 est une vue de dessus d'une installation de fabrication de récipients stériles comportant un four selon l'invention pourvu de moyens de stérilisation par ultraviolets pour la décontamination de la surface externe du col et du corps des préformes et, en amont du four, d'un poste de stérilisation pour réaliser simultanément dans le four la décontamination de la paroi interne de la préforme.

Dans la description, on utilisera à titre non limitatif les expressions telles que "amont" et "aval", "supérieur" et "inférieur", "intérieur" et "extérieur" et les orientations longitudinale, verticale et transversale en référence aux trièdres (L, V, T) représentés sur les figures et aux définitions données dans la description.

Dans la suite de la description, des éléments similaires ou identiques seront désignés par les mêmes références.

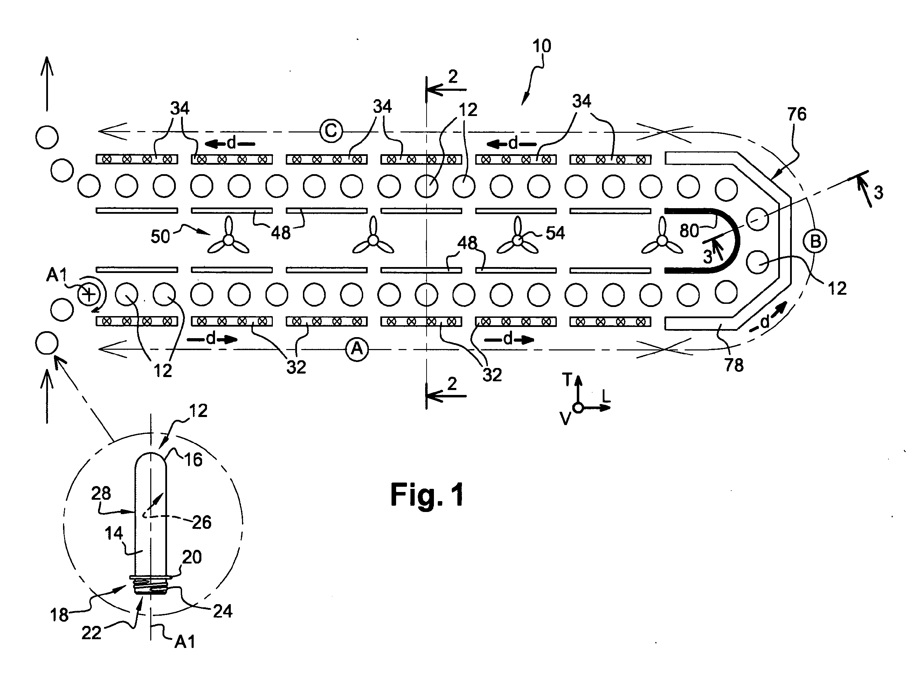

Sur la figure 1, on a représenté un exemple de réalisation d'un four 10 de conditionnement thermique de préformes, en particulier de préformes en matière plastique ou thermoplastique, telle que du Polyéthylènetéréphtalate (PET).

La figure 1 représente, sur une vue de détail, une préforme 12 destinée à la fabrication d'un récipient à corps creux, tel qu'une bouteille, un flacon etc., notamment par soufflage ou par étirage- soufflage.

Par définition, le terme "préforme" désigne dans la suite de la présente description aussi bien la préforme initiale obtenue par injection de matière plastique et utilisée dans des procédés de fabrication d'un récipient à une seule étape de soufflage aboutissant directement au récipient final que le récipient intermédiaire dans le cas des procédés à plusieurs étapes de soufflage.

Les préformes 12 sont généralement réalisées selon un procédé de moulage par injection et sont par exemple moulées sur un autre lieu que celui où se trouve l'installation de fabrication des récipients.

Comme on peut mieux le voir sur la vue de détail, une préforme 12 présente généralement un corps 14 globalement tubulaire, de section cylindrique suivant un plan de coupe horizontal, ledit corps 14 s'étendant verticalement selon un axe principal A1, ici dans une position dite "col en bas".

La préforme 12 est fermée à son extrémité supérieure par un fond 16 sensiblement hémisphérique, qui correspond à la partie supérieure du corps de la préforme destinée à former le fond du récipient.

A l'opposé, la préforme 12 est ouverte à son extrémité inférieure qui est conformée en un col 18 qui possède déjà la forme définitive du col ou goulot du récipient.

La préforme 12 comporte à la jonction entre le corps 14 et le col 18, une collerette 20 qui s'étend radialement vers l'extérieur en saillie par rapport au corps 14 et au col 18.

Le col 18 délimite une ouverture annulaire 22 centrée sur l'axe A1 et il comporte sur sa surface externe un filetage 24 destiné à permettre la mise en place ultérieure de moyens de fermeture à vis du récipient, tels qu'un bouchon (non représenté).

Les principales parties de la préforme 12 considérées dans le cadre de la stérilisation sont respectivement la surface de la paroi interne 26, la surface externe 28 du corps 14 s'étendant verticalement du fond 16 à la collerette 20 du col 18.

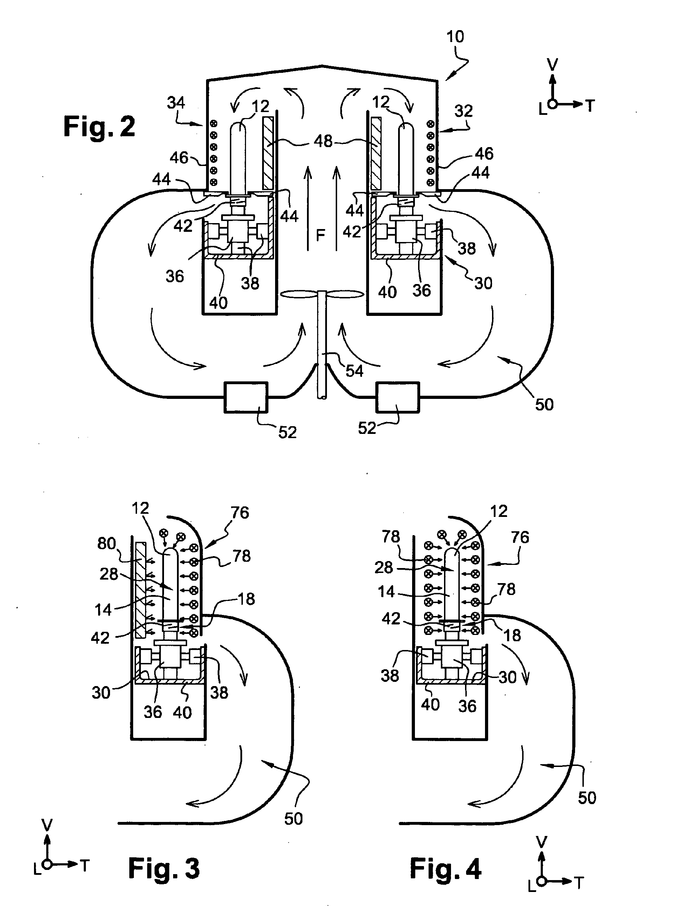

Comme représenté sur la figure 2, le four 10 comporte un dispositif de transport 30 destiné à convoyer successivement les préformes 12 d'amont en aval et qui est muni de moyens assurant la mise en rotation sur elle-même de chaque préforme autour de son axe vertical A1.

Les préformes 12 se déplacent ainsi depuis l'entrée du four 10 jusqu'à la sortie suivant le sens les flèches "d" représentées sur la figure 1 et parcourent d'amont en aval les différentes zones que comporte le four 10 à une vitesse de déplacement V tout en tournant simultanément chacune sur elle-même à une vitesse de rotation déterminée.

De manière connue, le four 10 comporte successivement une première zone A, dite de chauffage de pénétration, une deuxième zone intermédiaire B, dite de temporisation, et une troisième zone C de chauffage; dite de chauffage final, qui sont respectivement repérées en trait mixte sur la figure 1.

La première zone A de chauffage de pénétration comporte des premiers moyens de chauffage 32 émettant un rayonnement infrarouge IR1 qui est destiné à préchauffer le corps 14 de chaque préforme 12 jusqu'à une première température de consigne TC1.

De préférence, la première température de consigne TC1 correspondant ici à la température de préchauffage est comprise entre 50°C et 80°C, par exemple égale à 70°C.

La deuxième zone intermédiaire B de temporisation est interposée sur le trajet des préformes 12 entre la première zone A de chauffage et la troisième zone C de chauffage.

La deuxième zone B est parcourue par chaque préforme 12 en un laps de temps Δt déterminé, qui est notamment fonction de la vitesse de déplacement V des préformes 12 entraînées par le dispositif de transport 30.

En fonction des applications, le laps de temps Δt est par exemple compris entre 2 et 15 secondes, suivant la cadence de la machine, généralement de l'ordre de 5 secondes.

La temporisation effectuée dans la deuxième zone B, entre les première et troisième zones de chauffage, est destinée à permettre une répartition homogène dans l'ensemble du corps 14 de la chaleur transmise par les premiers moyens de chauffage 32.

La troisième zone C de chauffage de distribution comporte des seconds moyens de chauffage 34 émettant un rayonnement infrarouge IR2 qui est destiné à chauffer le corps 14 de chaque préforme 12 jusqu'à une deuxième température de consigne TC2.

De préférence, la deuxième température de consigne TC2 correspond ici à la température de chauffage est comprise entre 80°C et 130 °C, généralement de l'ordre de 90°C à 110°C.

La température de chauffage est notamment déterminée en fonction des caractéristiques de la préforme 12 telles que le matériau, l'épaisseur du corps 14, etc. et du récipient.

Les premiers moyens de chauffage 32 et/ou les seconds moyens de chauffage 34 sont par exemple constitués par des lampes à rayonnement infrarouge, généralement en forme de tube, qui en position de fonctionnement dans les zones A et C de chauffage s'étendent longitudinalement et sont montées les unes au dessus des autres dans des râteliers (non représentés).

Comme on peut le voir sur la figure 2, le dispositif de transport 30 des préformes 12 comporte des moyens de support 36 sur lesquels les préformes 12 sont montées en position "col en bas", soit le fond 16 dirigé vers le haut, et qui sont pourvus de moyens de guidage 38, par exemple à galets, engagés dans des chemins de roulement 40 complémentaires en forme de "U".

De préférence, les moyens de support 36 du dispositif de transport 30 comportent des moyens de préhension 42 du col 18 de chaque préforme 12, les moyens de préhension 42 comportant avantageusement un noyau interne (non représenté) qui s'étend verticalement vers le haut de manière à pénétrer axialement selon l'axe A1 à l'intérieur de l'ouverture 22 délimitée par le col 18.

Le four 10 comporte des moyens de protection 44, par exemple en forme de rail, formant des caches destinés à assurer une protection thermique du col 18 de chaque préforme 12 par rapport aux rayons infrarouges.

En effet, le col 18 de chaque préforme 12 présentant la forme définitive de celui du col ou goulot du récipient final, celui-ci ne doit pas être exposé au rayonnement infrarouge des moyens de chauffage 32, 34 du four pour éviter tout risque de déformation susceptible en particulier de compromettre ultérieurement, après remplissage, l'opération de bouchage du récipient.

Les moyens de protection 44 s'étendent horizontalement au dessus des supports 36 délimitant entre eux un passage calibré de manière à permettre respectivement au corps 14 de la préforme 12 s'étendant verticalement au dessus d'être chauffé et au col 18 s'étendant en dessous d'être au contraire protégé.

Avantageusement, les moyens de protection 44 sont refroidis par des moyens de refroidissement associés (non représentés).

Les première et troisième zones de chauffage A et C du four 10 s'étendent verticalement au dessus des moyens de protection 44 des cols 18 et comportent respectivement une paroi externe 46 et une paroi interne 48 entre lesquelles, transportées par le dispositif 30, défilent les corps 14 des préformes 12 à chauffer.

Dans chacune des zones de chauffage A et C du four 10, la paroi extérieure 46 comporte de préférence des panneaux isolants associés aux lampes infrarouges de chauffage 32 ou 34 qui sont superposées verticalement les unes au dessus des autres, tandis que la paroi intérieure 48 opposée est constituée de panneaux réflecteurs aptes à réfléchir les rayons infrarouges émis par les lampes 32, 34 en direction des préformes.

De préférence, la position transversale de chaque lampe de chauffage 32, 34 est susceptible d'être réglée sélectivement selon la direction transversale, notamment dans la troisième zone de chauffage C pour établir un profil de chauffe déterminé en faisant varier la distance entre chacune des lampes des seconds moyens de chauffage 34 et le corps 14 comme le fond 16 des préformes 12 en vis à vis.

Avantageusement, le four 10 comporte un dispositif 50 de refroidissement, dit superficiel, du corps 14 des préformes 12 qui est obtenu par circulation d'un flux d'air F autour des préformes tel qu'illustré par des flèches sur la figure 2.

Avantageusement, le dispositif 50 de circulation d'air de refroidissement comporte des moyens de filtration 52 destinés à filtrer le flux d'air de refroidissement mis en circulation dans le four 10 de manière à purifier l'air et éviter toute contamination des préformes par des germes, etc.

Avantageusement, l'air de refroidissement mis en circulation dans le four 10 est constitué par de l'air stérile ou présentant un degré de décontamination élevé.

De préférence, le dispositif 50 comporte des moyens de thermorégulation (non représentés) de la température de l'air.

Le four 10 est muni d'orifices d'aération pour permettre le passage du flux d'air F soufflé par l'intermédiaire de moyens à hélice 54 destinés à favoriser un chauffage homogène dans toute l'épaisseur de la préforme 12, notamment sans surchauffer la couche de matière superficielle de la surface externe 28 du corps 14 et du fond 16.

En effet, l'air permet d'évacuer la chaleur de convection provoquée par les moyens de chauffage 32, 34 et de favoriser la pénétration des rayonnements infrarouges IR1, IR2 émis dans l'épaisseur de la matière constituant la préforme 12.

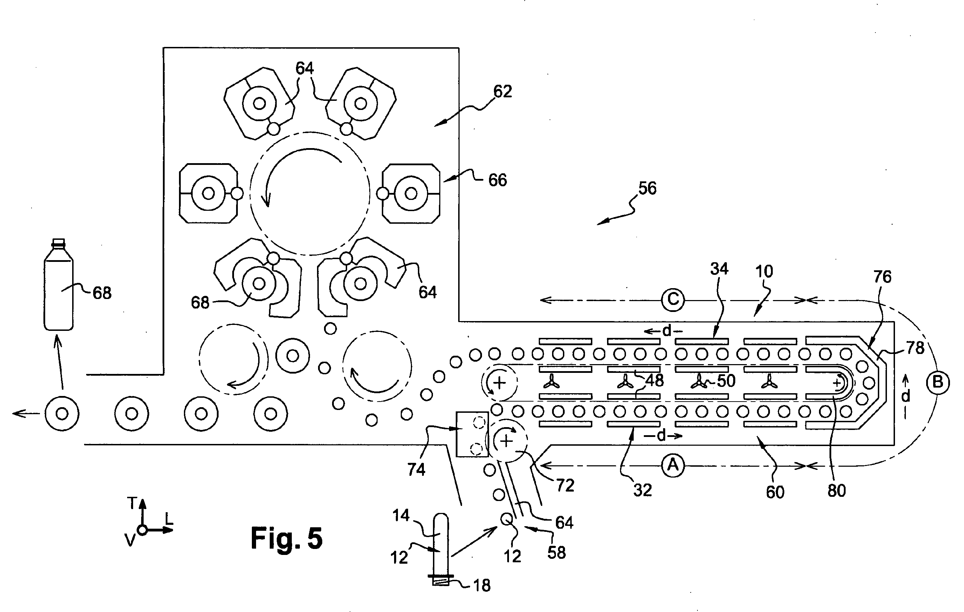

La figure 5 représente schématiquement une installation 56 pour la fabrication de récipients stériles, tels que des bouteilles, à partir de préformes 12 en matière thermoplastique du type de celle représentée à la figure 1.

Une telle installation 56 comporte principalement, suivant le sens de déplacement des préformes 12 de l'amont vers l'aval, un dispositif d'alimentation 58, un poste de conditionnement thermique 60 comportant au moins un four 10 et son dispositif de transport 30, un poste de moulage 62.

De manière connue, le four 10 du poste 60 est destiné à chauffer le corps 14 de chaque préforme en vue de permettre son moulage au poste de moulage 62 situé en aval.

Les moyens de chauffage 32 et 34 du four 10 assurent donc une première fonction consistant à chauffer les préformes 12 pour les ramollir, selon un profil de chauffe déterminé, en les portant à une température de moulage Tm fonction du matériau constitutif de la préforme.

Dans le cas d'une préforme réalisée en Polyéthylènetéréphtalate ou PET, la température de moulage Tm est d'environ 100°C.

La deuxième température de consigne TC2 des seconds moyens de chauffage 34 est égale à la température de moulage Tm, laquelle est supérieure à la température de transition vitreuse Tg du matériau constitutif de la préforme 12.

Le poste de moulage 62 comporte au moins un moule 64 et un dispositif de soufflage 66 associé pour transformer la préforme 12 en un récipient 68 à corps creux, ici une bouteille tel qu'illustré en détail à la figure 5 en sortie du poste 62 de l'installation 56.

Avantageusement, l'installation 56 comporte en aval du poste de moulage 62 transformant par soufflage chaque préforme 12 en une bouteille 68, un poste de remplissage et un poste de bouchage (non représentés).

Le dispositif d'alimentation 58 est agencé en entrée de l'installation 56 qu'il est destiné à alimenter en continu en préformes 12.

Un tel dispositif d'alimentation 58 comporte par exemple une trémie (non représentée) dans laquelle sont déversées en vrac des préformes 12 qui sont prélevées une par une à la base de la trémie pour être acheminées les unes à la suite des autres en position "col en haut".

Dans cette position "col en haut", les préformes 12 sont de préférence acheminées par gravité au moyen d'une glissière 70 inclinée, la glissière étant formée par deux rails parallèles écartés l'un de l'autre de manière à laisser passer verticalement le corps 14 des préformes 12 mais sur lesquels chaque préforme 12 repose par l'intermédiaire de sa collerette 20.

Des moyens préhenseurs aptes à saisir individuellement chaque préforme 12 sont par exemple disposés à l'extrémité de la glissière 70 et constitués par une roue de chargement 72.

La roue de chargement 72 est destinée à délivrer les préformes 12 en entrée du four 10 de conditionnement thermique
où elles sont prises en charge par le dispositif de transport 30.

Avantageusement, les préformes 12 sont convoyées à travers le four 10 par le dispositif de transport 30 dans une position opposée "col en bas", il est prévu des moyens de retournement (non représentés) des préformes entre la roue 72 et les supports 36 à noyau interne du dispositif 30.

Comme cela a été expliqué précédemment, il est nécessaire pour obtenir des récipients 68 stériles de procéder à une décontamination totale de la préforme 12.

Avantageusement, l'installation 56 comporte donc un poste de stérilisation 74 qui est par exemple disposé au dessus de la roue de chargement 72, en amont des moyens de retournement des préformes 12 et de l'entrée du four 10.

De préférence, le poste de stérilisation 74 (non représenté en détail) comporte un dispositif de projection de produit stérilisant pourvu d'au moins une buse apte à projeter, sous forme d'un jet de vapeur, un flux de produit stérilisant vers l'ouverture 22 du col 18 de chaque préforme 12, dont la température T0 est inférieure à la température de condensation Tc du produit stérilisant.

Grâce à ces moyens du poste de projection 74, on dépose par condensation sur au moins la paroi interne 26 de la préforme un film de buée de produit stérilisant sensiblement uniforme qui est destiné à stériliser la paroi interne 26.

Avantageusement, le produit stérilisant ou de stérilisation est constitué d'un composé contenant du peroxyde d'hydrogène H₂O₂ ou de peroxyde d'hydrogène H₂O₂ vaporisé.

La température d'activation Ta du peroxyde d'hydrogène H₂O₂ est d'environ soixante dix degrés Celsius (70°C).

Par conséquent, le produit stérilisant H₂O₂ se trouve activé dès l'entrée de la préforme 12 dans la première zone de préchauffage A du four 10 de sorte que la paroi interne 26 de la préforme 12 est stérilisée.

En effet, les premiers moyens de chauffage 32 à infrarouge du four 10 sont destinés à chauffer le corps 14 de la préforme 12 jusqu'à la première température de consigne TC1, laquelle est avantageusement supérieure ou égale à la température d'activation Ta.

De plus, les seconds moyens de chauffage 34 par infrarouge de la troisième zone C de chauffage du four 10 sont destinés à chauffer la préforme 12 à la deuxième température de consigné TC2 égale à la température de moulage Tm (de 90 à 130°C) qui est supérieure à la température d'évaporation Te d'un produit stérilisant, tel que l'H₂O₂.

Par conséquent, le produit stérilisant (H₂O₂) est automatiquement éliminé par évaporation lors du chauffage dans la zone C, de sorte que le récipient final 68 obtenu présente tout au plus quelques traces infimes de produit stérilisant conformément aux exigences légales en vigueur.

Avantageusement, les moyens de chauffage 32 et 34 du four 10 assurent donc, outre une première fonction conventionnelle de chauffage en vue du moulage, une deuxième fonction consistant à activer thermiquement le produit stérilisant par chauffage de manière à stériliser au moins la paroi interne 26 de la préforme 12 puis à éliminer par évaporation ledit produit préalablement activé afin d'en supprimer quasiment toutes traces dans le récipient final 68.

Avantageusement, la paroi interne 26 de la préforme 12 est en contact avec le film de buée de produit stérilisant déposé par condensation et qui est activé dès l'entrée dans le four 10 pendant une durée, dite décontamination interne, qui correspond au moins au laps de temps mis par une préforme pour parcourir successivement la première zone A.

En fonction de la température, la décontamination de la paroi interne 26 peut se poursuivre sur tout ou partie de la deuxième zone intermédiaire B, avant que le film de produit stérilisant ne s'évapore progressivement dans la troisième zone C.

La stérilisation de la paroi interne 26 de la préforme 12 étant avantageusement réalisée par voie chimique de la manière décrite ci-dessus, il est encore nécessaire pour fabriquer des récipients stériles présentant un degré de décontamination élevé, de procéder à la stérilisation de la surface externe 28 de la préforme 12.

En effet, comme cela a été expliqué précédemment, seule la stérilisation supplémentaire de la surface externe 28 du corps 14 de la préforme 12, comme de préférence celle du col 18, est susceptible de réduire, voir d'éliminer, les risques de contamination croisée selon lesquels des germes, bactéries ou spores etc. présents à l'extérieur de la préforme sont en particulier susceptibles d'être réintroduits à l'intérieur de la préforme 12, notamment par l'air en circulation au poste de moulage 62 lors de l'opération de soufflage.

Conformément à l'invention, le four 10 est **caractérisé en ce qu**'il comporte les premiers moyens de chauffage 32, les moyens de stérilisation 76 qui sont agencés dans la deuxième zone intermédiaire B de temporisation et qui sont aptes à émettre des rayons ultraviolets UV, et les seconds moyens de chauffage 34 de manière à stériliser au moins la surface externe 28 du corps 18 de la préforme 12 jusqu'au degré de décontamination déterminé.

Avantageusement, le procédé de stérilisation mis en oeuvre dans un four 10 selon l'invention est apte à stériliser au moins la surface externe 28 du corps 14 de la préforme 12 jusqu'à un degré de décontamination déterminé.

En effet, le procédé de stérilisation mis en oeuvre dans un four 10 selon l'invention comporte au moins les étapes successives suivantes :
- une première étape de décontamination par chauffage au cours de laquelle le corps 14 de la préforme 12 est préchauffé par les premiers moyens de chauffage 32 jusqu'à la première température de consigne TC1 ;
- une deuxième étape de décontamination par ultraviolets UV au cours de laquelle au moins la surface externe 28 du corps 14 de la préforme 12 est exposée pendant un laps de temps Δt déterminé aux rayons ultraviolets UV émis par des moyens de stérilisation 76 ; et
- une troisième étape de stérilisation par chauffage au cours de laquelle le corps 14 de la préforme 12 est chauffé par les seconds moyens de chauffage 34 jusqu'à une deuxième température de consigne TC2.

Avantageusement, un tel procédé de stérilisation est donc susceptible d'être mis en oeuvre dans un four 10 tel que le four 10 d'un poste de conditionnement thermique 60 d'une installation 56 de fabrication de récipients stériles à partir de préformes 12 en matières thermoplastiques destinées à être transformées par soufflage ou par étirage-soufflage.

Dans un four 10 selon l'inventions, la stérilisation d'au moins la surface externe 28 du corps 18 de la préforme 12 est obtenue par trois étapes consistant successivement à :
- chauffer ou préchauffer le corps 14 de la préforme 12 jusqu'à la première température de consigne TC1 correspondant à une première température donnée de décontamination ;
- irradier au moins le corps 14 de la préforme 12 par des rayons ultraviolets UV pendant une durée déterminée ; et
- chauffer le corps 14 de la préforme 12 jusqu'à la deuxième température de consigne TC2 correspondant à une deuxième température donnée de décontamination.

Avantageusement, les rayons ultraviolets UV émis par les moyens de stérilisation 76 dans la deuxième zone intermédiaire B de temporisation stérilisent l'air du flux d'air de refroidissement circulant autour des préformes 12.

Selon un exemple de réalisation représenté à la figure 3, le four 10 comporte dans la deuxième zone intermédiaire B de temporisation des moyens de stérilisation 76 par ultraviolets UV qui sont constitués par des lampes 78 à rayons ultraviolets UV et des moyens réfléchissants 80 associés, tels que des réflecteurs.

Avantageusement, les réflecteurs 80 sont agencés à l'intérieur du four 10 en vis-à-vis des lampes 78 à rayons ultraviolets UV de manière à réfléchir sélectivement les rayons ultraviolets UV émis en direction des préformes 12.

Les moyens de stérilisation 76 par ultraviolets formés par les lampes 78 à rayons ultraviolets UV et par les moyens réfléchissants 80 sont ici globalement agencés "en fer à cheval" suivant le trajet des préformes 12 dans la deuxième zone B et de part et d'autre de ces dernières qui défilent ainsi à la vitesse V et en tournant sur elle-même entre les lampes UV 78 et les réflecteurs 80.

Selon une variante de réalisation représentée à la figure 4, les réflecteurs 80 agencés à l'intérieur sont remplacés par une paroi pourvue de lampes 78 à ultraviolets UV de manière que la deuxième zone intermédiaire B de temporisation du four 10 comporte des moyens supplémentaires d'émission d'ultraviolets UV pour stériliser les préformes 12.

Avantageusement, une telle variante permet d'augmenter globalement le rayonnement ultraviolet UV émis et donc la quantité de radiations reçues par la surface externe 28 du corps 14 et par le col 18 de la préforme 12 de manière à accroître le degré final de décontamination, pour une même vitesse V de déplacement des préformes 12.

De plus, les moyens de stérilisation 76 selon cette variante permettent de maintenir un degré de décontamination élevé même lorsque la vitesse V augmentant le laps de temps Δt diminue.

En effet, le laps de temps Δt correspond à la durée d'exposition de la surface externe 28 du corps 14 et du col 18 de la préforme 12 aux rayons ultraviolets UV.

La température moyenne dans la deuxième zone B de temporisation est de l'ordre de 40 à 50°C de sorte que les moyens de protection 44 des cols 18 y sont avantageusement supprimés.

Avantageusement, les moyens de stérilisation 76 tels que les lampes 78 et/ou les réflecteurs 80 sont disposés de manière que le col 18 aussi soit irradié par les rayons ultraviolets UV.

Les lampes 78 des moyens de stérilisation 76 sont constituées par des lampes de type standard, par exemple en forme de tube, qui présentent à une température environnante de l'ordre de 50°C dans la deuxième zone B intermédiaire un fonctionnement fiable et une durée de vie normale.

Avantageusement, le dispositif de transport 30 comporte les moyens de préhension 42 du col 18 de chaque préforme 12 munis du noyau interne pénétrant axialement à l'intérieur de l'ouverture 22 du col 18 de la préforme 12 de sorte que la surface externe du col 18 de la préforme 12 est intégralement irradiée par les rayons ultraviolets UV émis par les moyens de stérilisation 76.

Le degré de décontamination de la surface externe du col 18 de la préforme 12 est donc au moins égal à 2,5 Log.

Pour déterminer le degré final de décontamination, des essais ont été effectués avec des préformes 12 contaminées par des spores du bacille "*Atrophaeus 9372*", utilisé ici comme référent, et dont les résultats sont détaillés ci-après.

En effet, les spores de germes de ce bacille sont aujourd'hui considérés comme référent dans le monde pour déterminer le niveau de stérilité ou d'asepsie, et cela tant par des organismes (FDA, ...) que par les industriels, en particulier dans un secteur comme l'agro-alimentaire.

En réalisant uniquement une étape de stérilisation thermique de la surface externe 28 du corps 14 de la préforme 12 par chauffage avec des rayons infrarouges dans un four 10, le degré de décontamination obtenu est relativement faible, généralement inférieur à 0,5 Log.

De même, en réalisant uniquement une opération de stérilisation de la surface externe 28 du corps 14 de la préforme 12 par irradiation avec des rayons ultraviolets UV pendant une durée déterminée, le degré de décontamination obtenu est alors compris entre 2 Log et 3 Log, par exemple de l'ordre de 2,5 Log.

La durée d'irradiation par ultraviolets UV des préformes 12 correspond avantageusement au laps de temps déterminé Δt mis par chaque préforme 12 pour parcourir la deuxième zone B de temporisation d'un four donné, chaque préforme 12 étant entraînée à travers le four 10 par le dispositif de transport 30 à une vitesse de déplacement V déterminée qui est fonction du type de four et des applications.

A titre de comparaison, les essais réalisés ont démontré qu'en réalisant consécutivement une étape de stérilisation par ultraviolets UV puis une étape de stérilisation thermique par infrarouge ou inversement une étape de stérilisation thermique par infrarouge puis une étape de stérilisation par ultraviolets UV, le degré de décontamination obtenu restait globalement similaire à celui obtenu auparavant avec la seule étape de stérilisation par ultraviolets UV, c'est-à-dire de l'ordre de 2,5 Log.

Par conséquent, les résultats obtenus démontrent clairement qu'une unique étape de stérilisation par chauffage, notamment par infrarouge, ne permet pas d'accroître le degré de décontamination au delà du degré obtenu avec la seule stérilisation par ultraviolets UV.

Cependant et de manière surprenante, on obtient avec la combinaison des trois étapes successives mises en oeuvre dans un four 10 selon l'invention un degré de décontamination aussi inattendu que particulièrement élevé.

Par comparaison, un degré final de décontamination de l'ordre de 5 Log à 6 Log a été obtenu lors de ces essais, soit un niveau de stérilisation de la surface externe 28 du corps 14 de la préforme 12 qui est de plus de deux fois supérieur à celui obtenu précédemment en réalisant consécutivement seulement deux étapes de stérilisation par chauffage infrarouge et par irradiation par ultraviolets UV ou inversement.

La mise en oeuvre du procédé dans un four 10 selon l'invention comportant les moyens de stérilisation 76 s'effectue au moins selon les étapes successives de décontamination décrites précédemment pour stériliser au moins la paroi externe 28 du corps 14 de la préforme 12.

Par conséquent, les moyens de chauffage 32 et 34 du four 10 assurent encore une troisième fonction consistant à favoriser la décontamination de la préforme 12 en combinant les effets stérilisants du chauffage par infrarouge et de l'irradiation par ultraviolets UV pour obtenir un degré final de décontamination particulièrement élevé.

Avantageusement, le degré final de décontamination de la surface externe 28 du corps 14 est obtenu uniquement par la combinaison de ces trois étapes de stérilisation qui, réalisées consécutivement, vont permettre successivement d'éliminer au moins une première partie des germes, bactéries ou spores etc. présents sur la surface externe 28 du corps 14 de la préforme 12 qui sont détruits et/ou altérés par la chaleur des rayons infrarouges IR1, et qui dès lors, à tout le moins fragilisés, présentent une vulnérabilité accrue grâce à laquelle les ultraviolets UV vont réaliser une destruction ou une altération d'autres germes etc. supplémentaires, la majorité des germes etc. subsistants sur la surface externe 28 du corps 14 de la préforme 12 sont alors détruits par la forte chaleur des rayons infrarouges IR2.

En effet, la présence de certains germes, bactéries ou spores etc. sur la surface externe 28 du corps 14 de la préforme 12 en sortie de la deuxième zone B, qui n'ont été à ce stade qu'altérés, ne permet pas de garantir de manière irréversible le degré de décontamination, ce n'est qu'après le passage dans la troisième zone C pour être exposés aux rayons infrarouges IR2 que leur destruction est obtenue de manière irréversible.

Avantageusement, le procédé de stérilisation comporte une étape préliminaire de stérilisation de la paroi interne 24 de la préforme 12 qui, précédant la première étape de préchauffage, consiste à projeter sous la forme d'un jet de vapeur un flux de produit stérilisant vers l'ouverture 22 du col 18 de chaque préforme 12 se trouvant à une température T0 inférieure à la température de condensation Tc du produit stérilisant de manière à provoquer, sur la paroi interne 24 de la préforme 12, le dépôt par condensation d'un film de buée de produit stérilisant sensiblement uniforme.

Le film uniforme de buée de produit stérilisant condensé qui est ensuite activé thermiquement par les premiers moyens de chauffage 32 du four, puis éliminé par évaporation par les seconds moyens de chauffage 34, permet de stériliser la paroi interne 26 avec un degré de décontamination d'au moins 3 Log.

Grâce à l'invention, il est possible à partir de préformes 12 stérilisées et conditionnées thermiquement dans un four 10 selon l'invention de fabriquer des récipients stériles présentant un degré final de décontamination particulièrement élevé et cela tant sur la paroi interne 26 de la préforme 12 que sur la surface externe 28 du corps 14 de la préforme 12.

En effet, la deuxième fonction des moyens de chauffage 32 et 34 du four 10 est d'activer thermiquement puis d'éliminer par évaporation le produit stérilisant déposée par condensation.

Avantageusement, la première température de préchauffage TC1 est donc supérieure ou égale à la température d'activation Ta du produit stérilisant de manière à stériliser la paroi interne 26 de la préforme 12 tandis que la deuxième température de chauffage TC2 est supérieure à la température d'évaporation Te du produit stérilisant de manière à éliminer par évaporation le produit stérilisant préalablement activé thermiquement.

Avantageusement, le procédé de stérilisation selon l'invention peut être mis en oeuvre de manière particulièrement simple, fiable et économique, notamment dans une installation de fabrication de récipients stériles du type de celle décrite et représentée à la figure 5.

En effet, un four de conditionnement 10 conforme aux enseignements de l'invention décrits précédemment comporte successivement :
- la première zone A, dite de chauffage de pénétration, comportant les premiers moyens de chauffage 32 par rayonnement infrarouge IR1 destinés à préchauffer le corps 14 de chaque préforme 12 jusqu'à ladite première température de consigne TC1 ;
- la deuxième zone intermédiaire B de temporisation qui, interposée sur le trajet des préformes entre la première zone A de chauffage et la troisième zone C de chauffage, est parcourue par chaque préforme en un laps de temps Δt déterminé ; et
- la troisième zone C de chauffage de distribution, comportant les seconds moyens de chauffage 34 par rayonnement infrarouge IR2 destinés à chauffer le corps 14 de chaque préforme 12, selon un profil de chauffe déterminé, jusqu'à ladite deuxième température de consigne TC2.

Par conséquent, les modifications structurelles à apporter dans un tel four 10 existant pour y implanter les moyens de stérilisation 76 par ultraviolets UV sont minimes et parfaitement localisées dans la deuxième zone B de temporisation.

Les rayonnements infrarouges des premiers et seconds moyens de chauffage 32, 34 ont avantageusement une triple action, à savoir de chauffage de la préforme 12 en vue de la transformation par soufflage ou par étirage-soufflage en un récipients creux stériles, de stérilisation (ou décontamination) de la paroi interne 26 par activation et évaporation du produit stérilisant et, en combinaison avec l'irradiation par ultraviolets UV, de stérilisation dite thermique de la surface externe 28 du corps 14.

## Revendications

1. Four (10) de conditionnement thermique de préformes en matière thermoplastique, notamment destiné à équiper une installation de fabrication de récipients stériles, du type comportant un dispositif de transport (30) des préformes (12) qui sont mises en rotation sur elle-même et convoyées successivement entre au moins :
- une première zone A, dite de chauffage de pénétration, comportant des premiers moyens de chauffage (32) par rayonnement infrarouge (IR1) destinés à préchauffer le corps (14) de chaque préforme (12) jusqu'à une première température de consigne (TC1) ;
- une deuxième zone intermédiaire B, dite de temporisation qui, interposée sur le trajet des préformes (12) entre la première zone A de chauffage et une troisième zone C de chauffage, est parcourue par chaque préforme (12) en un laps de temps (Δt) déterminé ;
- la troisième zone C, dite de chauffage de distribution, comportant des seconds moyens de chauffage (34) par rayonnement infrarouge (IR2) destinés à chauffer le corps (14) de chaque préforme (12), selon un profil de chauffe déterminé, jusqu'à une deuxième température de consigne (TC2),
**caractérisé en ce que** le four (10) comporte des moyens de stérilisation (76, 78, 80) par émission de rayons ultraviolets (UV) qui sont agencés dans la deuxième zone intermédiaire B de temporisation de manière à stériliser au moins la surface externe (28) du corps (14) de la préforme (12) jusqu'à un degré de décontamination déterminé.

2. Four selon la revendication 1, **caractérisé en ce que** le four (10) comporte des moyens réfléchissants (80), tels que des réflecteurs, qui sont agencés dans la deuxième zone intermédiaire B de temporisation en vis-à-vis des moyens de stérilisation (76) par ultraviolets (UV), tels que des lampes (78), de manière à réfléchir sélectivement les rayons ultraviolets (UV) émis en direction des préformes (12) circulant entre les moyens de stérilisation (UV) et les moyens réfléchissants (80).

3. Four selon l'une des revendications 1 ou 2, du type comportant un dispositif de refroidissement (50) des préformes (12) par circulation d'un flux d'air (F) autour des préformes (12),
**caractérisé en ce que** les rayons ultraviolets (UV) émis par les moyens de stérilisation (76) dans la deuxième zone intermédiaire B de temporisation stérilisent l'air circulant autour des préformes (12).

4. Four selon l'une quelconque des revendications précédentes, **caractérisé en ce que** le dispositif de transport (30) comporte des moyens de préhension (42) du col (18) de chaque préforme (12) comportant au moins un noyau interne qui pénètre axialement à l'intérieur du col (18) de la préforme (12) de manière que les rayons ultraviolets (UV) émis par les moyens de stérilisation (76) irradient intégralement le col (18) de la préforme (12).

5. Installation (56) pour la fabrication de récipients stériles (68) à partir d'une préforme (12) en matière thermoplastique décontaminée, comportant au moins :
- un dispositif d'alimentation (58) en préformes ;
- un poste de conditionnement thermique (60) comportant un dispositif de transport (30) de préformes à travers au moins un four (10) destiné à chauffer chaque préforme (12) en vue de son moulage ; et
- un poste de moulage (62) destiné à transformer chaque préforme (12) en un récipient (68) à corps creux,
**caractérisée en ce que** le four (10) de conditionnement thermique comporte :
- des premiers moyens de chauffage (32) destinés à décontaminer au moins la surface externe (28) du corps (14) de la préforme (12) en préchauffant le corps (14) de chaque préforme (12) jusqu'à une première température de consigne (TC1) ;
- des moyens de stérilisation (76) par émission de rayons ultraviolets (UV) destinés à décontaminer au moins la surface externe (28) du corps (14) de la préforme (12) par irradiation de la préforme (12) exposée pendant un laps de temps (Δt) déterminé aux rayons ultraviolets (UV) émis par les moyens de stérilisation (76) ; et
- des seconds moyens de chauffage (34) destinés à décontaminer au moins la surface externe (28) du corps (14) de la préforme (12) en chauffant le corps (14) de chaque préforme (12); jusqu'à une deuxième température de consigne (TC2),
de manière à stériliser au moins la surface externe (28) du corps (14) de la préforme (12) jusqu'à un degré de décontamination déterminé.

6. Installation (56) selon la revendication 5, **caractérisée en ce qu'**elle comporte, en amont du four (10) de conditionnement thermique, un poste de stérilisation (74) comportant un dispositif de projection de produit stérilisant pourvus d'au moins une buse apte à projeter, sous forme d'un jet de vapeur, un flux de produit stérilisant vers l'ouverture (22) du col (18) de chaque préforme (12), dont la température (T0) est inférieure à la température de condensation (Tc) du produit stérilisant, de manière à déposer par condensation sur au moins la paroi interne (26) de la préforme (12) un film de buée de produit stérilisant sensiblement uniforme destiné à stériliser au moins la paroi interne (26).

7. Installation (56) selon la revendication 6, **caractérisé en ce que** la première température de préchauffage (TC1) et/ou la deuxième température de chauffage (TC2) du corps (14) de la préforme (12) dans le four (10) sont supérieures ou égales à la température d'activation (Ta) du produit stérilisant de manière à stériliser la paroi interne (26) de la préforme (12) en activant le produit stérilisant par chauffage.

8. Installation (56) selon la revendication 7, **caractérisé en ce que** la première température de préchauffage (TC1) et/ou la deuxième température de chauffage (TC2) du corps de la préforme (12) dans le four (10) sont supérieures à la température d'évaporation (Te) du produit stérilisant de manière à éliminer par évaporation le produit stérilisant activé par chauffage.

## Claims

1. An oven (10) for heat-conditioning of thermoplastic preforms, particularly intended for equipping an installation for manufacture of sterile receptacles, comprising a device for transport (30) of the preforms (12) which are rotated around their own axes and successively conveyed within at least:
- one first zone A, which may be called a deep-heating zone, comprising first means for heating (32) by infrared radiation (IR1) intended to preheat the body (14) of each preform (12) up to a first setpoint temperature (TC1);
- a second zone B, which is an intermediate zone, and which may be called a residence zone, through which zone, which is interposed in the path of the preforms (12) between the first zone A, which is a heating zone, and a third zone C, which is a heating zone, each preform (12) passes, during a defined period ( t);
- the third zone C, which may be called a distributed-heating zone, comprising second means for heating (34) by infrared radiation (IR2), intended to heat the body (14) of each preform (12), in accordance with a defined heater profile, up to a second setpoint temperature (TC2),
**characterized in that** the oven (10) comprises means for sterilization (76, 78, 80) by emission of ultraviolet (UV) radiation, arranged in the second zone B, which is an intermediate residence zone, in such a way as to sterilize at least the external surface (28) of the body (14) of the preform (12) up to a defined degree of decontamination.

2. The oven as claimed in claim 1, **characterized in that** the oven (10) comprises means for reflection (80), such as reflectors, arranged in the second zone B, which is an intermediate residence zone, opposite to the means for sterilization (76) by ultraviolet (UV) radiation, such as lamps (78), in such a way as to reflect selectively the ultraviolet (UV) radiation emitted in the direction of the preforms (12) passing between the means for sterilization (UV) and the means for reflection (80).

3. The oven according to claim 1 or 2, comprising a device for the cooling (50) of the preforms (12) by passage of a current of air (F) around the preforms (12), **characterized in that** the ultraviolet (UV) radiation emitted by the means for sterilization (76) in the second zone B, which is an intermediate residence zone, sterilizes the air circulating around the preforms (12).

4. The oven as claimed in any of the preceding claims, **characterized in that** the device for transport (30) comprises means for grasping (42) the neck (18) of each preform (12) comprising at least one internal core which penetrates axially into the interior of the neck (18) of the preform (12) in such a way that the ultraviolet (UV) radiation emitted by the means for sterilization (76) irradiates entirely the neck (18) of the preform (12).

5. An installation (56) for the manufacture of sterile receptacles (68) starting from a decontaminated thermoplastic preform (12), comprising at least:
- one device for feed (58) of preforms;
- one unit for heat-conditioning (60) comprising a device for transport (30) of preforms through at least one oven (10) intended to heat each preform (12) with the aim of molding of the same; and
- a unit for molding (62) intended to convert each preform (12) to a hollow-bodied receptacle (68),
**characterized in that** the oven (10) for heat-conditioning comprises:
- first means for heating (32) intended to decontaminate at least the external surface (28) of the body (14) of the preform (12) by preheating the body (14) of each preform (12) up to a first setpoint temperature (TC1);
- means for sterilization (76) by emission of ultraviolet (UV) radiation intended to decontaminate at least the external surface (28) of the body (14) of the preform (12) by irradiation of the preform (12) exposed during a defined period (Δt) to ultraviolet (UV) radiation emitted by the means for sterilization (76); and
- second means for heating (34) intended to decontaminate at least the external surface (28) of the body (14) of the preform (12) by heating the body (14) of each preform (12) up to a second setpoint temperature (TC2),
in such a way as to sterilize at least the external surface (28) of the body (14) of the preform (12) up to a defined degree of decontamination.

6. The installation (56) as claimed in claim 5, **characterized in that** it comprises, upstream of the oven (10) for heat-conditioning, a unit for sterilization (74) comprising a device for projection of sterilizing product provided with at least one nozzle capable of projecting, in the form of a vapor jet, a flow of sterilizing product toward the aperture (22) of the neck (18) of each preform (12), the temperature (T0) of which is lower than the temperature of condensation (Tc) of the sterilizing product, in such a way as to deposit by condensation on at least the internal wall (26) of the preform (12) an approximately uniform condensation film of sterilizing product, intended to sterilize at least the internal wall (26).

7. The installation (56) as claimed in claim 6, **characterized in that** the first temperature of preheating (TC1) and/or the second temperature of heating (TC2) of the body (14) of the preform (12) in the oven (10) are higher than or equal to the temperature of activation (Ta) of the sterilizing product, in such a way as to sterilize the internal wall (26) of the preform (12) by activation of the sterilizing product by heating.

8. The installation (56) as claimed in claim 7, **characterized in that** the first temperature of preheating (TC1) and/or the second temperature of heating (TC2) of the body of the preform (12) in the oven (10) are higher than the temperature of evaporation (Te) of the sterilizing product, in such a way as to remove by evaporation the sterilizing product activated by heating.

## Patentansprüche

1. Ofen (10) zur thermischen Aufbereitung von Vorformen aus thermoplastischem Material, die insbesondere dazu bestimmt sind, eine Anlage zur Herstellung von sterilen Gefäßen auszustatten, mit einer Transportvorrichtung (30) für Vorformen (12), welche zum Drehen um sich selbst gebracht werden, und nacheinander befördert werden, zwischen mindestens:
- einer ersten Zone A der sogenannten Durchdringungs-Erwärmung, welche erste Erwärmungselemente (32) durch Infrarotstrahlung (IR1) besitzt, mittels derer der Körper (14) jeder Vorform (12) bis auf eine erste Solltemperatur (TC1) erwärmt werden soll;
- einer zweiten Zwischenzone B der sogenannten Verzögerung, die - eingeschoben auf dem Weg der Vorformen (12) zwischen der ersten Aufwärmzone A und einer dritten Aufwärmzone C - von jeder Vorform (12) in einem festgelegten Zeitraum (Δt) durchlaufen wird;
- einer dritten Zone C der sogenannten Verteilungs-Erwärmung, welche zweite Erwärmungselemente (34) durch Infrarotstrahlung (IR2) besitzt, mittels derer der Körper (14) jeder Vorform (12) gemäß einem festgelegten Erwärmungsprofil bis auf eine zweite Solltemperatur (TC2) erwärmt werden soll,
**dadurch gekennzeichnet, dass** der Ofen (10) Mittel zur Sterilisierung (76, 78, 80) durch Aussenden von Ultraviolettstrahlen (UV-Strahlen) besitzt, welche in der zweiten Zwischenzone B der Verzögerung angeordnet sind, um mindestens die Außenseite (28) des Körpers (14) der Vorform (12) bis zu einem bestimmten Grad der Dekontamination zu sterilisieren.

2. Ofen (10) nach Anspruch 1, **dadurch gekennzeichnet, dass** der Ofen (10) reflektierende Elemente (80), wie Reflektoren, besitzt, die in der zweiten Zwischenzone B der Verzögerung gegenüber den Mitteln (76) zur Sterilisierung durch Ultraviolettstrahlen (UV-Strahlen) angeordnet sind, wie beispielsweise Lampen (78), um die Ultraviolettstrahlen (UV-Strahlen), die in Richtung der Vorformen (12) - welche zwischen den Mitteln zur Sterilisierung (UV) und den reflektierenden Elementen (80) zirkulieren - ausgesendet werden, selektiv zu reflektieren.

3. Ofen (10) nach einem der Ansprüche 1 oder 2 mit einer Vorrichtung zur Kühlung (50) der Vorformen (12) durch Zirkulation eines Luftstromes (F) um die Vorformen (12) herum, **dadurch gekennzeichnet, dass** die Ultraviolettstrahlen (UV-Strahlen), die von den Mitteln zur Sterilisierung (76) in der zweiten Zwischenzone B der Verzögerung ausgesendet werden, die Luft, welche um die Vorformen (12) herum zirkuliert, sterilisieren.

4. Ofen (10) nach einem der vorhergehenden Ansprüche, **dadurch gekennzeichnet, dass** die Transportvorrichtung (30), Elemente zum Greifen (42) des Halses (18) jeder Vorform (12) besitzt, welche mindestens einen inneren Kern aufweisen, der axial in das Innere des Halses (18) der Vorform (12) eindringt, so dass die Ultraviolettstrahlen (UV-Strahlen), die von den Mitteln zur Sterilisation (76) ausgesendet werden, den gesamten Hals (18) der Vorform (12) bestrahlen.

5. Anlage (56) zur Herstellung von sterilen Gefäßen (68) aus einer Vorform (12) aus dekontaminiertem, thermoplastischem Material, die mindestens folgendes aufweist:
eine Vorform-Zuführvorrichtung (58);
eine Station zur thermischen Aufbereitung (60), die eine Vorform -Transportvorrichtung (30) durch mindestens einen Ofen (10) besitzt, der dazu dient, jede Vorform (12) im Hinblick auf ihre Abformung zu erhitzen; und
Abform-Station (62), die dazu dient, jede Vorform (12) in ein Gefäß mit hohlem Körper zu verwandeln,
**dadurch gekennzeichnet, dass** der Ofen (10) zur thermischen Aufbereitung folgendes aufweist:
- erste Erwärmungselemente (32), um mindestens die Außenseite (28) des Körpers (14) der Vorform (12) zu dekontaminieren, indem der Körper (14) jeder Vorform (12) bis zu einer ersten Solltemperatur (TC1) erwärmt wird;
- Mittel zur Sterilisierung (76) durch Aussenden von Ultraviolettstrahlen (UV-Strahlen), die dazu bestimmt sind, mindestens die Außenseite des Körpers (14) der Vorform (12) durch Bestrahlen der exponierten Vorform (12) während eines bestimmten Zeitraums (Δt) mit Ultraviolettstrahlen (UV-Strahlen), die durch die Mittel zur Sterilisation (76) ausgesendet werden, zu dekontaminieren; und
- zweite Erwärmungselemente (32), die dazu bestimmt sind, mindestens die Außenseite des Körpers (14) der Vorform (12) durch Erwärmen des Körpers (14) jeder Vorform (12) bis auf eine zweite Solltemperatur (TC2) zu dekontaminieren, um mindestens die Außenseite des Körpers (14) der Vorform (12) bis zu einem festgelegten Dekontaminationsgrad zu sterilisieren.

6. Anlage (56) nach Anspruch 5, **dadurch gekennzeichnet, dass** sie dem Ofen (10) zur thermischen Aufbereitung vorgelagert eine Station zur Sterilisierung (74) mit einer Vorrichtung zum Aufsprühen des Sterilisationsproduktes besitzt, die mit mindestens einer Düse versehen ist, welche in Form eines Dampfstrahls einen Strom des Sterilisationsproduktes in die Richtung der Öffnung (22) des Halses (18) jeder Vorform (12) sprüht, dessen Temperatur (T0) niedriger ist als die Kondensationstemperatur (Tc) des Sterilisationsproduktes, so dass auf mindestens der Innenwand (26) der Vorform (12) durch Kondensation eine im wesentlichen gleichmäßige, dünne Dampfschicht des Sterilisationsproduktes aufgebracht wird, die dazu dient, mindestens die Innenwand (26) zu sterilisieren.

7. Anlage (56) nach Anspruch 6, **dadurch gekennzeichnet, dass** die erste Erwärmungstemperatur (TC1) und / oder die zweite Erwärmungstemperatur (TC2) des Körpers (14) der Vorform (12) in dem Ofen (10) höher als die Aktivierungstemperatur (Ta) oder gleich der Aktivierungstemperatur (Ta) des Sterilisationsproduktes ist, so dass die Innenwand (26) der Vorform (12) sterilisiert wird, indem das Sterilisationsprodukt durch Erwärmen aktiviert wird.

8. Anlage (56) nach Anspruch 7, **dadurch gekennzeichnet, dass** die erste Erwärmungstemperatur (TC1) und / oder die zweite Erwärmungstemperatur (TC2) des Körpers (14) der Vorform (12) in dem Ofen (10) höher ist als die Verdampfungstemperatur (Te) des Sterilisationsproduktes, um das durch die Erwärmung aktivierte Sterilisationsprodukt durch Verdampfen zu beseitigen.
